# EUROPEAN PATENT SPECIFICATION

(11) **EP 1 853 540 B1**
(45) Date of publication and mention of the grant of the patent: **04.04.2018**
(21) Application number: 06708431.9
(22) Date of filing: 21.02.2006
(51) Int. Cl.: C07C 41/30

(54) **PROCESS**
VERFAHREN
PROCÉDÉ

(30) Priority: 28.02.2005 GB 0504079
(43) Date of publication of application: 14.11.2007
(73) Proprietor: Johnson Matthey Davy Technologies Limited, London EC4A 4AB (GB)
(72) Inventor: ROBERTS, Simon Darby The Technology Center, Stockton-on-Tees TS17 6PY (GB)
(74) Representative: Bown, Mark Richard
(86) International application number: PCT/EP2006/060156
(87) International publication number: WO 2006/089900

(56) References cited:
- DE-A1- 19 904 900
- US-A- 4 694 111

## Description

The present invention relates to a process for alkoxyalkylation of an aromatic substrate. More particularly it relates to a process for the selective alkoxyalkylation of an aromatic substrate. Still more particularly, it relates to a process for the selective alkoxymethylation of an aromatic substrate. In particular it relates to the dialkoxymethylation of an aromatic substrate. Most particularly it relates to the alkoxymethylation of naphthalene to afford a 2,6-disubstituted naphthalene. In a preferred arrangement, the present invention relates to a process for the production of 2,6-di(alkoxymethyl)naphthalene. In a second embodiment the present invention relates to a process for the production of polymers and in particular polyesters such as polyethylene naphthalate.

It is well known that alkylated polycyclics such as 2,6-dialkylnaphthalenes are valuable materials as they are precursors to polyesters such as polyethylene naphthalate. Polyethylene naphthalate has superior strength and barrier properties over other polyesters, such as polyethylene terephthalate, and is used to make fibres, films and packaging materials. In view of the improved properties obtained with polyethylene naphthalate substantial research effort has been carried out with a view to obtaining an economical manufacturing route to the product and/or its precursors.

Polyethylene naphthalate is generally produced either by esterification of 2,6-naphthalenedicarboxylic acid or by transesterification of dimethyl 2,6-naphthalenedicarboxylate. The 2,6-naphthalenedicarboxylic acid and 2,6-naphthalenedicarboxylate starting materials for these reactions are generally produced from 2,6-dialkylnaphthalene.

However, the production of the desired 2,6-dialkylnaphthalene starting materials has particular difficulties since there are ten dialkylnaphthalene isomers.

There are a number of known methods for the production of 2,6-dimethylnaphthalene. For example, as described in US 4963248, 2,6-dimethylnaphthalene can be recovered from fractions during kerosene reformation or, as described in European Chemical

News, P. 30, 28.09.1992 and in US 6121501, it can be recovered from fractions of FCC oil. Whilst these processes provide the desired compound, because of the small differences in the boiling points of the various dimethylnaphthalene isomers complicated selective adsorption, separation and crystallisation procedures have to be used. The separation of the 2,6- and 2,7-isomers are particularly difficult. These isomers can also form a eutectic mixture.

It has also been suggested that isomerisation technology can be used to convert mixed dimethylnaphthalene isomers into the desired 2,6-dimethylnaphthalene. Examples of potentially suitable isomerisation technologies are described in US 4777312, US 5495060, US 6015930 and US 6018087. However as described in US 6057487 only the 1,5- and 1,6- isomers are readily isomerised.

Effort has also focussed on selectively producing the 2,6-isomer or the di-isomer 'triad' (i.e. 2,6-, 1,5- and 1,6-dimethylnaphthalene). In particular, efforts have been made to provide processes which avoid the co-production of the 2,7-isomer. One such process involves a series of reactions starting from o-xylene and butadiene to prepare 1,5-dimethylnaphthalene which is further isomerised to the desired isomer over a zeolite catalyst. Suitable processes are described in US 4990717, US 5073670, US 5118892, US 5030781 and US 5012024. However, this four step route is far from ideal since a number of secondary reactions occur which necessitates purification steps to be carried out for each intermediate.

A more direct process to 2,6-dimethylnaphthalene has been described in US 4795847. This process starts from naphthalene or methylnaphthalene and comprises methylation with a suitable methylating agent in the presence of a zeolite catalyst. Zeolite catalysts have been shown to hold a number of advantages over homogeneous Friedel-Crafts type catalysts. These benefits include the ease of catalyst separation, waste, corrosion and toxicity minimisation, as well as the ability to affect selectivity. As reported in US 5001295 the selection of the appropriate zeolite for the methylation of naphthalene or 2-methylnaphthalene can produce a mixture that is rich in the desired 2,6-isomers. However due to the near identical molecular diameters of the 2,6- and 2,7- dimethylnaphthalene isomers the product mixture only weakly favours the desired isomer.

Efforts have been made to enrich 2,6-dimethylnaphthalene in the product by transalkylating unwanted dimethylnaphthalene isomers with naphthalene over a zeolite to produce 2-methylnaphthalene which can then be selectively alkylated to 2,6-dimethylnaphthalene. One process of this type is described in US 6011190. Further isomerisation to 2,6-dimethylnaphthalene then occurs followed by separation from the remaining dimethylnaphthalene isomers and other products.

To utilise the shape-selective properties of zeolites, bulkier substituents have been added to the naphthalene substrate such that the difference in the critical molecular dimension between the 2,6- and 2,7-isomer is maximised. As described in WO 90/03960, US 5900519 and US 4950824, isopropylation of naphthalene or 2-isopropylnaphthalene over a zeolite catalyst can produce mixtures rich in 2,6-diisopropylnaphthalene.

Substantial research effort has been made to improve the 2,6/2,7 ratio by careful optimisation of the catalyst properties and process conditions. A number of alternative substituents have been attached to naphthalene and its derivatives, although none thus far have met the desired criteria for selectivity, ease of subsequent oxidation (generally the bulkier the alkyl substituent the more difficult the oxidation), and atom efficiency. In this connection it is noted that four carbon atoms would be lost during oxidation of 2,6-diisopropylnaphthalene to 2,6-naphthalenedicarboxylic acid. Examples of processes using alternative substituents include that described in US 4873386 in which an ethyl substituent is used, that described in Org. Biomol. Chem., 2003, 1552-1559 in which the substituent used is tert-butyl and that described in US 5210350 in which dicyclohexyl is used. US 5210355, US 5235115 and US 5321182 describe processes in which combinations of substituents are used.

Even once the production of the 2,6-dialkylnaphthalene starting material has been achieved, problems are encountered in the oxidation, purification, esterification and/or further refinement to high-purity 2,6-naphthalenedicarboxylic acid or 2,6-naphthalenedicarboxylate.

One method for the production of 2,6-naphthalenedicarboxylic acid is described in US 3870754, US 3856855, US 4709088, US 4716245, US 4794195, US 4950786, US 5144066, US 5183933 and WO 04/015003. In this process the 2,6-dialkylnaphthalenes are oxidised in acetic acid in the presence of a cobalt/manganese catalyst and a bromide promoter. However, due to the corrosive nature of the chemicals used in this reaction, it is necessary to fabricate the reactor vessels from high cost materials such as titanium. A further disadvantage of the process is that 2,6-naphthalenedicarboxylic acid obtained requires substantial purification to remove the various impurities. These impurities include trimellitic acid, 6-formyl-2-naphthoic acid and brominated naphthalene compounds. In addition, insoluble heavy metal complexes (cobalt and manganese) are formed, particularly with trimellitic acid, which can result in downstream process fouling. The purification of 2,6- naphthalenedicarboxylic acid is further complicated as the acid has low solubility in most solvents and decomposes at its melting point.

Crude 2,6-naphthalenedicarboxylic acid may be esterified to 2,6-naphthalenedicarboxylate with methanol as purification of the ester, whilst still complex, offers certain advantages. Examples of esterification processes can be found in US 6013831 and US 6211398. US 5095135 and US 5254719 teach that sulphuric acid is an effective catalyst for the esterification reaction and reacts with the heavy metal impurities to form soluble sulphate salts. However, corrosion issues and waste sulphate disposal are further problems encountered with this process.

In order to achieve high-purity 2,6-naphthalenedicarboxylate, dissolution in a suitable solvent, typically an aromatic hydrocarbon, and further insolubles separation is required, followed by recrystallisation and distillation steps. Thus the six common steps required to convert 2,6-dialkyl naphthalene to 2,6-naphthalenedicarboxylate which is suitable for polyethylene naphthalate production are 1) oxidation 2) esterification 3) dissolution 4) separation 5) recrystallisation and 6) distillation. A further process step involves the recovery and recycling of the expensive oxidation catalyst metals.

It is therefore desirable to provide a process which provides a 2,6-disubstituted naphthalene from naphthalene which can readily be converted to polyethylene naphthalate. This can be achieved where at least one of the substituents on the naphthalene is an alkoxyalkyl, particularly an alkoxymethyl, group. 2,6-di(alkoxymethyl)naphthalene offers significant advantages over 2,6-dialkylnaphthalene in the production of polyester as it is more readily oxidised, thus milder conditions can be employed in the oxidation step of the polyester production process. The use of milder conditions will overcome the problems of low yield and the requirement for expensive purification steps which are noted with prior art processes.

It is also desirable to provide a process for the selective alkoxyalkylation of other unsubstituted aromatic substrates. US4694111 describes the methoxyalkylation of phenol by reacting the phenol with a lower acetal or ketal at an elevated temperature in the presence of an H-ZDM-5 zeolite as catalyst. DE 19904900 also discloses the reaction production of alkoxyalkylation of mono-substituted aromatic compounds in the presence of a zeolite as catalyst. However, there is no teaching of a process for the alkoxyalkylation of unsubstituted aromatic substrates.

Thus according to the present invention there is provided a process for alkoxyalkylation of an unsubstituted aromatic substrate said process comprising reacting the aromatic substrate with a dialkoxyalkane in the presence of a catalyst.

The alkoxyalkylation is preferably a process selective for a desired substitution. The selectivity may be achieved by any suitable means. In one arrangement, a shape selective catalyst such as a zeolite may be used.

Where the catalyst is a zeolite any suitable zeolite may be used. The choice of zeolite may influence the product selectivity as the geometry of the framework type will affect the position of aromatic substitution. The reaction can be catalysed over a broad acidity range. Wholly acidic zeolites and materials partially exchanged with basic cations may be used. Zeolite mordenite has been found to offer particular advantages.

The SiO₂/Al₂O₃ ratio of the zeolite may influence activity and selectivity. It is believed that acidic mordenite catalysts having higher SiO₂/Al₂O₃ ratios offer improved selectivity for the desired isomer.

Any suitable physical form of catalyst may be used. Thus powders and extrudates may be used.

In an alternative arrangement a non-shape selective catalyst may be used in a shape selective environment. For example, a homogenous catalyst such as a Lewis acid catalyst may be used if held within a shape selective host. Any suitable Lewis acid catalyst may be used. Suitable catalysts include AlCl₃.

The aromatic substrate may be any substrate that is susceptible to electophilic substitution. The aromatic substrate may be a mono or polycyclic aromatic compound and will preferably be a mono or polycyclic hydrocarbon, although heteroaryl compounds may also be used. Any suitable heteroaryl compound may be used.

Where the substrate is a polycyclic aromatic compound it may have fused rings or rings that are connected via a bond, for example a bi-phenyl.

In a particularly preferred arrangement, the disubstituted product is substituted such that the substituents are spaced to the maximum amount. For example, where the aromatic substrate on which the reaction is carried out is a monocyclic aromatic compound, the substituents will be located in the para position. Similarly, where the aromatic substrate is a polycyclic compound they will be spaced to the greatest extent. Thus where the substrate is naphthalene, the substituents will be located at the 2,6 position. Where the substrate is a biphenyl, the substituents will generally be located at the 4,4' position.

Where the product of the process of the present invention is for use as a polyethylene naphthalate precursor, the aromatic substituent will preferably be naphthalene

Any suitable dialkoxyalkane may be used. Dialkoxymethanes are particularly preferred. Suitable dialkoxymethanes include dimethoxymethane and diethoxymethane. The alkoxymethylating agent may be chosen to influence the product selectivity. Without wishing to be bound by any theory, it is believed that where a bulkier substituent is formed, selectivity will be improved.

The reaction may be carried out in the liquid phase or in the gas phase. The process may be operated as a batch or a continuous process. The reaction may be carried out in a fixed bed reactor, a fluidised bed reactor or a slurry reactor. Whatever type of reactor is used, the process may be carried out in the presence of a shape selective catalyst or host.

A liquid-phase continuous process utilising a fixed bed of catalyst or of shape selective host offers some advantages. It will be understood that where the catalyst is shape selective, the shape selective host will generally be the catalyst.

The reaction may be carried out in the presence or absence of solvent. Suitable solvents include non-polar hydrocarbons such as hexane or cyclohexane. Acetone, trichloromethane or diethyl ether may also be used. If no solvent is used, the reactants may act as solvent.

Any suitable temperature may be used. It is desirable to use a temperature which provides a good yield of substituted product whilst minimising by-product formation. It is believed that if the temperature is too low, the yield of the reaction is low and if the temperature is too high, the yield of desired product is diminished due to competing side reactions or the further reaction of the product to undesirables.

Temperatures in the range of from about 100°C to about 200°C are useful with temperatures of from about 110°C to 170°C being preferred.

The process of the present invention may be carried out under self-generated pressure which is typically less than about 150 psig (1136kPa). This is particularly suitable where the process is carried out as a batch process. Pressures of from about 5 barg (500kPa) to about 50 barg (500kPa) may offer certain advantages.

Any suitable reaction time may be used. It is believed that if the reaction time is too short the yield of desirable product may be reduced. However, if the reaction time is too long, the selectivity may be reduced as by-products, undesirable isomers, and breakdown products may be obtained. Reaction times in the region of from about 1 hour to about 72 hours may be used. Preferably the reaction time is from about 1 hour to about 40 hours with times in the region of from about 4 hours to about 18 hours being preferred.

According to a second aspect of the present invention there is provided a process for the production of a carboxylic acid or carboxylate comprising:
producing a di(alkoxyalkyl)aromatic compound in accordance with the above first aspect of the present invention; and
oxidising the alkoxyalkyl side chains to the corresponding carboxylic acid or carboxylate.

The carboxylic acid or carboxylate produced by the process of the above second aspect may be polymerised in a process for the production of polyesters.

The carboxylic acid or carboxylate produced by the process of the above second aspect may be used in a process for the production of polyesters.

Where the aromatic compound is naphthalene, the process will produce polyethylene naphthalate.

The process may also be used for the production of other polyesters. The process of the third aspect of the present invention may be used to produce polyalkylene terephthalates, polyalkylene phthalates, polyalkylene naphthalates, and polyalkene biphenylates.

Thus, where the aromatic substrate is benzene, the process may produce polyethylene terephthalate. Similarly, biphenyl can be used in the process to provide a route to speciality polyesters which are based on a biphenyl backbone.

This process offers an improved process since the substituent groups are in a favourable oxidation state prior to the oxidation step and thus milder oxidation conditions can be employed.

The present invention will now be described with reference to the following examples. Unless stated, all examples were carried out in 100 ml stirred autoclaves, operating under self-generated pressure.

In the examples below, the numbers expressed as percentages have been rounded to the nearest whole number, except numbers which are less than one which are expressed to 1 decimal place. Isomer ratios are direct comparisons of gas chromatograph peak areas and expressed to 1 decimal place.

### Example 1

The methoxymethylation of 10 mmol naphthalene is carried out with 80 mmol dimethoxymethane over 4g H+ zeolite catalyst with 60ml cyclohexane as solvent. The catalyst used is Beta SiO₂/Al₂O₃ 25. The reaction was carried out at 150°C. After 18 hours there was found to be 59 mol% naphthalene conversion, giving yields of 37 mol% (methoxymethyl)naphthalene and 6 mol% di(methoxymethyl)naphthalene and <0.1 mol% of the undesirable by-product methylene-bis-naphthalenes. The 2/1 ratio was 1.9 and the 2,6/2,7 ratio was 2.9.

### Example 2

Example 1 was repeated with the catalyst being replaced with a sodium exchanged beta zeolite SiO₂/Al₂O₃ 25. After 18 hours there was found to be 46 mol% naphthalene conversion, giving yields of 18 mol% (methoxymethyl)naphthalene and 5 mol% di(methoxymethyl)naphthalene and <0.1 mol% of the undesirable by-product methylene-bis-naphthalenes. The 2/1 ratio was 1.6 and the 2,6/2,7 substitution ratio was 6.0.

### Example 3

Example 1 was repeated with the catalyst being replaced with a acidic zeolite mordenite SiO₂/Al₂O₃ 20. After 18 hours there was found to be 13 mol% naphthalene conversion, giving yields of 4 mol% (methoxymethyl)naphthalene and 0 mol% di(methoxymethyl)naphthalene and <0.1 mol% of the undesirable by-product methylene-bis-naphthalenes. The 2/1 ratio was 12.9.

### Examples 4 to 8

Example 1 was repeated utilising different solvents. The results are set out in Table 1. In each example, 60 ml of the solvent was used.

**Table 1**

| **Example No** | 4 | 5 | 6 | 7 | 8 |
|---|---|---|---|---|---|
| **Solvent** | Cyclohexane | Hexane | Acetone | Trichlorom ethane | Dimethyl ether |
| **Naphthalene conversion (mol%)** | 59 | 47 | 52 | 54 | 37 |
| **(methoxymethyl)naphthalene (mol%)** | 37 | 23 | 39 | 37 | 8 |
| **2/1 ratio** | 1.9 | 1.8 | 2.5 | 2.6 | 3.5 |
| **di(methoxymethyl)naphthalene (mol%)** | 6 | 1 | 6 | 3 | 1 |
| **2,6/2,7 ratio** | 2.9 | 2,6- only | 5.2 | 3.7 | 2,6- only |

### Examples 9 to 13

Example 1 was repeated at a variety of temperatures. The results are set out in Table 2.

**Table 2**

| **Example No:** | 9 | 10 | 11 | 12 | 13 |
|---|---|---|---|---|---|
| **Temperature (°C)** | 115 | 120 | 125 | 130 | 150 |
| **naphthalene conversion (mol%)** | 36 | 34 | 47 | 54 | 59 |
| **(methoxymethyl)naphthalene (mol%)** | 19 | 20 | 20 | 18 | 37 |
| **2**/**1 ratio** | 2.9 | 3 | 2.6 | 2.1 | 1.9 |
| **di(methoxymethyl)naphthalene (mol%)** | 2 | 2 | 2 | 3 | 6 |
| **2,6/2,7 ratio** | 2.7 | 2.4 | 2.7 | 2.0 | 2.9 |

### Examples 14 to 19

Example I was repeated at a temperature of 120°C and at a range of reaction times. The results are set out in Table 3.

**Table 3**

| **Example No** | 14 | 15 | 16 | 17 | 18 | 19 |
|---|---|---|---|---|---|---|
| **Time (hours)** | 4 | 6 | 9 | 18 | 36 | 72 |
| **naphthalene conversion (mol%)** | 41 | 45 | 46 | 44 | 46 | 51 |
| **(methoxymethyl)naphthalene (mol%)** | 18 | 16 | 20 | 20 | 21 | 21 |
| **2/1 ratio** | 2.6 | 3 | 2.5 | 3 | 2.5 | 2.2 |
| **di(methoxymethyl)naphthalene (mol%)** | 1 | 2 | 2 | 2 | 2 | 2 |
| **2**,**6**/**2**,**7 ratio** | 2,6- only | 2.1 | 2.5 | 2.4 | 2.0 | 1.5 |

### Examples 20 to 23

Example 1 was repeated at a temperature of 120°C and at a reaction time of 4 hours with varying amounts of catalyst. The results are set out in Table 4.

**Table 4**

| **Example No** | 20 | 21 | 22 | 23 |
|---|---|---|---|---|
| **Amount of catalyst (g)** | 1 | 2 | 4 | 8 |
| **Naphthalene conversion (mol%)** | 22 | 27 | 41 | 58 |
| **(methoxymethyl)naphthalene (mol%)** | 17 | 16 | 18 | 18 |
| **2/1 ratio** | 3.2 | 2.8 | 2.6 | 2.7 |
| **di(methoxymethyl)naphthalene (mol%)** | 0 | 0 | 1 | 5 |
| **2,6/2,7 ratio** | - | - | 2,6- only | 7.3 |

Whilst selectivity was noted to be low with a low amount of catalyst, it is believed that longer reaction time would give the desired product. Without wishing to be bound by any theory, it is believed that in the short time period and low catalyst amount, only mono substitution occurs.

### Examples 24 and 25

Example 1 was repeated for a reaction time of 4 hours and at a temperature of 120°C, with 758 mmol dimethoxymethane and with a catalyst having differing SiO₂/Al₂O₃ ratios. The results are set out in Table 5.

**Table 5**

| **Example No** | 24 | 25 |
|---|---|---|
| **Catalyst** | Beta SiO₂/Al₂O₃ 25 | Beta SiO₂/Al₂O₃ 75 |
| **Naphthalene conversion (mol%)** | 82 | 67 |
| **(methoxymethyl)naphthalene (mol%)** | 38 | 34 |
| **2/1 ratio** | 2.2 | 1.7 |
| **Di(methoxymethyl)naphthalene (mol%)** | 18 | 14 |
| **2,6/2,7 ratio** | 5.8 | 5.0 |

### Examples 26 and 27

Examples 24 and 25 were repeated with a mordenite catalyst having differing SiO₂/Al₂O₃ ratios. The results are set out in Table 6.

**Table 6**

| **Example No** | 26 | 27 |
|---|---|---|
| **Catalyst** | Mordenite SiO₂/Al₂O₃ 20 | Mordenite SiO₂/Al₂O₃ 90 |
| **naphthalene conversion (mol%)** | 52 | 86 |
| **(methoxymethyl)naphthalene (mol%)** | 40 | 44 |
| **2/1 ratio** | 3.7 | 4.0 |
| **di(methoxymethyl)naphthalene (mol%)** | 8 | 28 |
| **2,6/2,7 ratio** | 11.6 | 11.7 |

### Example 28

Example 27 was repeated with the dimethoxymethane being replaced with 535 mmol diethoxymethane as reagent. The results are compared in Table 7.

**Table 7**

| **Example No** | 27 | 28 |
|---|---|---|
| **Reagent** | dimethoxymethane 758 mmol, 67 ml | diethoxymethane 535 mmol, 67 ml |
| **naphthalene conversion (mol%)** | 86 | 27 |
| **(alkoxymethyl)naphthalene (mol%)** | 44 | 22 |
| **2/1 ratio** | 4.0 | 11.2 |
| **di(alkoxymethyl)naphthalene (mol%)** | 28 | 0 |
| **2,6/2,7 ratio** | 11.7 | - |

### Examples 29 and 30

10 mmol of 2-(methoxymethyl)naphthalene was reacted with 758 mmol dimethoxymethane over 4g powdered catalyst for 4 hours at a temperature of 120°C. The results are set out in Table 8.

**Table 8**

| **Example No** | 29 | 30 |
|---|---|---|
| **Catalyst** | Beta SiO₂/Al₂O₃ 25 | Mordenite SiO₂/Al₂O₃ 90 |
| **2-(methoxymethyl)naphthalene conversion (mol%)** | 55 | 63 |
| **Di(methoxymethyl)naphthalene (mol%)** | 52 | 57 |
| **2,6/2,7 ratio** | 8.6 | 9.7 |

### Examples 31 and 32

Example 27 was repeated using benzene as a starting material with different catalysts. The results are set out in Table 9.

**Table 9**

| **Example No** | 34 | 35 |
|---|---|---|
| **Catalyst** | ZSM-5 (SiO₂/Al₂O₃ 50) | Mordenite (SiO₂/Al₂O₃ 90) |
| **Benzene conversion (mol %)** | 31 | 65 |
| **Benzyl methyl ether (mol %)** | 19 | 25 |
| **di(methoxymethyl) benzene (mol %)** | 6 | 16 |
| ***Para*/*ortho* ratio** | 71.5 | 14.4 |

### Example 33

Example 27 was repeated using biphenyl as starting material. The only mono-substituted isomer obtained was the 4-(methoxymethyl) biphenyl and the only desired
di-substituted isomer was the desired 4,4'-(dimethoxymethyl)biphenyl. The results are set out in Table 10.

**Table 10**

| **Example No** | 36 |
|---|---|
| **Catalyst** | Mordenite (SiO₂/Al₂O₃ 90) |
| **biphenyl conversion (mol %)** | 30 |
| **4-(methoxymethyl) biphenyl (mol %)** | 22 |
| **4,4'-(dimethoxymethyl) biphenyl (mol %)** | 7 |
| **Selectivity to desirables (mol %)** | 94.9 |

### Example 34

In order to assess the operation of the invention at a larger scale with an extrudate form of the catalyst (Mordenite (SiO₂/Al₂O₃ 200)), a 1 litre batch reactor with spinning catalyst basket was used. Methoxymethylation of 89 mmol naphthalene was carried out with 600 ml of dimethoxymethane over 45 g of extrudate catalyst (20% binder) at 110 °C for 2 hrs. The results are set out in Table 11.

**Table 11**

| **Example No** | 37 |
|---|---|
| **Catalyst** | Mordenite (SiO₂/Al₂O₃ 200) |
| **naphthalene conversion (mol %)** | 66 |
| **(methoxymethyl)naphthalene (mol %)** | 40 |
| **2/1 ratio** | 6.2 |
| **di(methoxymethyl)naphthalene (mol %)** | 19 |
| **2,6/2,7 ratio** | 11.3 |

### Example 35

This example was carried out in order to demonstrate the reaction with reduced reagent at increased pressure without wishing to be bound by any theory, it is believed that pressure increases the concentration of dimethoxymethane in solution. Methoxymethylation of 10 mmol naphthalene was carried out with 379 mmol of dimethoxymethane over 4 g of Mordenite SiO₂/Al₂O₃ 200 at 110 °C, 100 psig (791 kPa) N₂ for 1 hr. The results are set out in Table 12.

**Table 12**

| **Example No** | 38 |
|---|---|
| **reagent** | Dimethoxymethane 379 mmol, 33.5 ml |
| **naphthalene conversion (mol %)** | 76 |
| **(methoxymethyl)naphthalene (mol %)** | 43 |
| **2/1 ratio** | 5.5 |
| **di(methoxymethyl)naphthalene (mol %)** | 22 |
| **2,6/2,7 ratio** | 10.8 |

### Example 36

An example was carried out to show the reaction in a fixed bed operation with Mordenite SiO₂/Al₂O₃ 90 extrudate catalyst (50 ml). A feed composition of 1/18.95 mol ratio of napththalene/dimethoxymethane was used. The product composition set out in Table 13 was achieved after 63.5 hrs online with a 3.3 h residence time at 120°C and 10 barg system pressure (N₂). The results are set out in Table 13.

**Table 13**

| **Example No** | 39 |
|---|---|
| **Naphthalene/dimethoxymethane Feed ratio (mol)** | 1/18.95 |
| **naphthalene conversion (mol %)** | 50 |
| **(methoxymethyl)naphthalene (mol %)** | 31 |
| **2/1 ratio** | 3.2 |
| **di(methoxymethyl)naphthalene (mol %)** | 7 |
| **2**,**6**/**2**,**7 ratio** | 6.6 |

### Examples 37 to 39

The reaction was carried out in a continuous stirred tank operation with Mordenite SiO₂/Al₂O₃ 90 extrudate catalyst 45 g in a 1 litre autoclave equipped with spinning basket. The reactor was operated on level control at 600 ml. The feed composition had a 1/75.8 mol ratio of naphthalene/dimethoxymethane. The product compositions set out in Table 14 were achieved with a 5 h reactor residence time at 110°C.

**Table 14**

| **Example No** | 40 | 41 | 42 |
|---|---|---|---|
| **Time (hours)** | 6 | 12 | 18 |
| **naphthalene conversion (mol%)** | 63 | 61 | 63 |
| **(methoxymethyl)naphthalene (mol %)** | 32 | 30 | 31 |
| 2/1 **ratio** | 4.6 | 4.2 | 4.1 |
| **di(methoxymethyl)naphthalene (mol %)** | 21 | 20 | 20 |
| **2,6/2,7 ratio** | 10.1 | 9.4 | 9.3 |

### Examples 40 and 41

Benzyl methyl ether was methoxymethylated with different catalysts. The experimental conditions are identical to those in Example 27. The results are set out in Table 15.

**Table 15**

| **Example No** | 43 | 44 |
|---|---|---|
| **Catalyst** | ZSM-5 (SiO₂/Al₂O₃ 50) | Mordenite (SiO₂/Al₂O₃ 90) |
| **Benzyl methyl ether conversion (mol %)** | 24 | 47 |
| **di(methoxymethyl) benzene (mol %)** | 20 | 34 |
| ***Para*/*ortho* ratio** | 123.8 | 17.0 |

## Claims

1. A process for the dialkoxyalkylation of an unsubstituted aromatic substrate, said process comprising reacting the aromatic substrate with a dialkoxyalkane in the presence of a catalyst.

2. A process according to Claim 1 wherein the alkoxyalkylation is selective for a desired substitution.

3. A process according to Claim 1 or 2 wherein the catalyst is a shape selective catalyst.

4. A process according to Claim 3 wherein the shape selective catalyst is a zeolite.

5. A process according to Claim 4 wherein the zeolite is mordenite.

6. A process according to Claim 1 or 2 wherein the catalyst is non-shape selective and is used in a shape selective environment.

7. A process according to Claim 6 wherein the shape selective environment includes the use of shape selective host.

8. A process according to any one of Claims 1 to 7 wherein the aromatic substrate is a mono or polycyclic hydrocarbon compound.

9. A process according to any one of Claims 1 to 7 wherein the aromatic substrate is a mono or polycyclic heteroaryl compound.

10. A process according to any one of Claims 1 to 7 wherein the substrate is benzene or a bi-phenyl.

11. A process according to any one of Claims 1 to 9 wherein the substrate is a polycyclic aromatic compound having fused rings.

12. A process according to any one of Claims 1 to 8 wherein the aromatic substrate is naphthalene.

13. A process according to any one of Claims 1 to 12 wherein the dialkoxyalkane is a dialkoxymethane.

14. A process according to Claim 13 wherein the dialkoxymethane is dimethoxymethane or diethoxymethane.

15. A process according to any one of Claims 1 to 14 wherein the process is a liquid-phase continuous system utilising a fixed bed, a fluidised bed or a slurry reactor.

16. A process according to Claim 15 wherein the process is carried out in the presence of a shape selective catalyst or host.

17. A process according to any one of Claims 1 to 16 wherein a solvent is used.

18. A process according to Claim 17 wherein the solvent is hexane, cyclohexane, acetone, trichloromethane or diethyl ether.

19. A process according to any one of Claims 1 to 18 wherein the process is carried out a temperature of from 100°C to 200°C.

20. A process according to any one of Claims 1 to 18 wherein the process is carried out a temperature of from 110°C to 170°C.

21. A process according to any one of Claims 1 to 20 wherein the process is carried out at a pressure of from 5 barg (500kPa) to 50 barg (500kPa).

22. A process according to any one of Claims 1 to 21 wherein the process is carried out for a period of from 4 hours to 18 hours.

23. A process for the production of a carboxylic acid or carboxylate comprising:
producing a di(alkoxyalkyl)aromatic compound in accordance with the process of any one of Claims 1 to 22; and
oxidising the alkoxyalkyl side chains to the corresponding carboxylic acid or carboxylate.

## Patentansprüche

1. Verfahren zum Dialkoxyalkylieren eines unsubstituierten aromatischen Substrats, wobei das Verfahren das Umsetzen des aromatischen Substrats mit einem Dialkoxyalkan in der Gegenwart eines Katalysators umfasst.

2. Verfahren nach Anspruch 1, wobei das Alkoxyalkylieren für eine gewünschte Substitution selektiv ist.

3. Verfahren nach Anspruch 1 oder 2, wobei der Katalysator ein formselektiver Katalysator ist.

4. Verfahren nach Anspruch 3, wobei der formselektive Katalysator ein Zeolith ist.

5. Verfahren nach Anspruch 4, wobei der Zeolith Mordenit ist.

6. Verfahren nach Anspruch 1 oder 2, wobei der Katalysator nicht formselektiv ist und in einer formselektiven Umgebung verwendet wird.

7. Verfahren nach Anspruch 6, wobei die formselektive Umgebung die Verwendung eines formselektiven Hosts enthält.

8. Verfahren nach einem der Ansprüche 1 bis 7, wobei das aromatische Substrat eine mono- oder polycyclische Kohlenwasserstoffverbindung ist.

9. Verfahren nach einem der Ansprüche 1 bis 7, wobei das aromatische Substrat eine mono- oder polycyclische Heteroarylverbindung ist.

10. Verfahren nach einem der Ansprüche 1 bis 7, wobei das Substrat Benzol oder ein Biphenyl ist.

11. Verfahren nach einem der Ansprüche 1 bis 9, wobei das Substrat eine polycyclische aromatische Verbindung mit kondensierten Ringen ist.

12. Verfahren nach einem der Ansprüche 1 bis 8, wobei das aromatische Substrat Naphthalin ist.

13. Verfahren nach einem der Ansprüche 1 bis 12, wobei das Dialkoxyalkan ein Dialkoxymethan ist.

14. Verfahren nach Anspruch 13, wobei das Dialkoxymethan Dimethoxymethan oder Diethoxymethan ist.

15. Verfahren nach einem der Ansprüche 1 bis 14, wobei das Verfahren ein kontinuierliches Flüssigphasensystem ist, das ein Festbett, ein Wirbelbett oder einen Suspensionsreaktor verwendet.

16. Verfahren nach Anspruch 15, wobei das Verfahren in der Gegenwart eines formselektiven Katalysators oder Wirts durchgeführt wird.

17. Verfahren nach einem der Ansprüche 1 bis 16, wobei ein Lösungsmittel verwendet wird.

18. Verfahren nach Anspruch 17, wobei das Lösungmittel Hexan, Cyclohexan, Aceton, Trichlormethan oder Diethylether ist.

19. Verfahren nach einem der Ansprüche 1 bis 18, wobei das Verfahren bei einer Temperatur von 100 °C bis 200 °C ausgeführt wird.

20. Verfahren nach einem der Ansprüche 1 bis 18, wobei das Verfahren bei einer Temperatur von 110 °C bis 170 °C ausgeführt wird.

21. Verfahren nach einem der Ansprüche 1 bis 20, wobei das Verfahren bei einem Druck von 5 barg (500 kPa) bis 50 barg (500 kPa) ausgeführt wird.

22. Verfahren nach einem der Ansprüche 1 bis 21, wobei das Verfahren über einen Zeitraum von 4 Stunden bis 18 Stunden ausgeführt wird.

23. Verfahren zum Herstellen einer Carbonsäure oder eines Carboxylats, Folgendes umfassend:
Herstellen einer aromatischen Di(akloxyalkyl)-Verbindung nach dem Verfahren nach einem der Ansprüche 1 bis 22; und
Oxidieren der Alkoxyalkylseitenketten an die/das entsprechende Carbonsäure oder Carboxylat.

## Revendications

1. Procédé de dialcoxyalkylation d'un substrat aromatique non substitué, ledit procédé comprenant la réaction du substrat aromatique avec un dialcoxyalcane en la présence d'un catalyseur.

2. Procédé selon la revendication 1 dans lequel l'alcoxyalkylation est sélective pour une substitution souhaitée.

3. Procédé selon la revendication 1 ou 2 dans lequel le catalyseur est un catalyseur sélectif vis-à-vis de la forme.

4. Procédé selon la revendication 3 dans lequel le catalyseur sélectif vis-à-vis de la forme est une zéolite.

5. Procédé selon la revendication 4 dans lequel la zéolite est la mordénite.

6. Procédé selon la revendication 1 ou 2 dans lequel le catalyseur est non sélectif vis-à-vis de la forme et est utilisé dans un environnement sélectif vis-à-vis de la forme.

7. Procédé selon la revendication 6 dans lequel l'environnement sélectif vis-à-vis de la forme comprend l'utilisation d'un hôte sélectif vis-à-vis de la forme.

8. Procédé selon l'une quelconque des revendications 1 à 7 dans lequel le substrat aromatique est un composé hydrocarboné mono ou polycyclique.

9. Procédé selon l'une quelconque des revendications 1 à 7 dans lequel le substrat aromatique est un composé hétéroaryle mono ou polycyclique.

10. Procédé selon l'une quelconque des revendications 1 à 7 dans lequel le substrat est le benzène ou un bi-phényle.

11. Procédé selon l'une quelconque des revendications 1 à 9 dans lequel le substrat est un composé aromatique polycyclique ayant des cycles condensés.

12. Procédé selon l'une quelconque des revendications 1 à 8 dans lequel le substrat aromatique est le naphtalène.

13. Procédé selon l'une quelconque des revendications 1 à 12 dans lequel le dialcoxyalcane est un dialcoxyméthane.

14. Procédé selon la revendication 13 dans lequel le dialcoxyméthane est le diméthoxyméthane ou le diéthoxyméthane.

15. Procédé selon l'une quelconque des revendications 1 à 14 dans lequel le procédé est un système continu en phase liquide utilisant un lit fixe, un lit fluidisé ou un réacteur en suspension.

16. Procédé selon la revendication 15 dans lequel le procédé est conduit en présence d'un catalyseur ou d'un hôte sélectif vis-à-vis de la forme.

17. Procédé selon l'une quelconque des revendications 1 à 16 dans lequel un solvant est utilisé.

18. Procédé selon la revendication 17 dans lequel le solvant est l'hexane, le cyclohexane, l'acétone, le trichlorométhane ou l'éther de diéthyle.

19. Procédé selon l'une quelconque des revendications 1 à 18 dans lequel le procédé est conduit à une température de 100°C à 200°C.

20. Procédé selon l'une quelconque des revendications 1 à 18 dans lequel le procédé est conduit à une température de 110°C à 170°C.

21. Procédé selon l'une quelconque des revendications 1 à 20 dans lequel le procédé est conduit à une pression de 5 bars (pression manométrique) (500 kPa) à 50 bars (pression manométrique) (500 kPa).

22. Procédé selon l'une quelconque des revendications 1 à 21 dans lequel le procédé est conduit sur une durée allant de 4 heures à 18 heures.

23. Procédé de production d'un acide carboxylique ou d'un carboxylate comprenant :
la production d'un composé di(alcoxyalkyl)aromatique selon le procédé selon l'une quelconque des revendications 1 à 22 ; et
l'oxydation des chaînes latérales alcoxyalkyle en l'acide carboxylique ou le carboxylate correspondant.
